**Europäisches Patentamt**

**European Patent Office**

**Office européen des brevets**

(19)

(11) Veröffentlichungsnummer : **0 065 929 B2**

(12) # NEUE EUROPÄISCHE PATENTSCHRIFT

(45) Veröffentlichungstag der neuen Patentschrift :
**28.07.93 Patentblatt 93/30**

(21) Anmeldenummer : **82730063.3**

(22) Anmeldetag : **06.05.82**

(51) Int. Cl.⁵ : **A61K 7/00, A61K 7/48**

---

(54) **Verfahren zur Herstellung eines kosmetischen Mittels.**

---

Die Akte enthält technische Angaben, die nach
dem Eingang der Anmeldung eingereicht
wurden und die nicht in dieser Patentschrift
enthalten sind.

(30) Priorität : **08.05.81 AT 2071/81**

(43) Veröffentlichungstag der Anmeldung :
**01.12.82 Patentblatt 82/48**

(45) Bekanntmachung des Hinweises auf die
Patenterteilung :
**10.09.86 Patentblatt 86/37**

(45) Bekanntmachung des Hinweises auf die
Entscheidung über den Einspruch :
**28.07.93 Patentblatt 93/30**

(84) Benannte Vertragsstaaten :
**AT BE CH DE FR IT LI LU NL SE**

(56) Entgegenhaltungen :
**FR-A- 1 603 602**

(56) Entgegenhaltungen :
**FR-A- 2 326 914
DERWENT JAPANESE PATENTS REPORT,
Band R. Nr. 47, 15. Januar 1971, Artikel Nr.
87444R, Derwent Publication Ltd., GB.
Deutsche Apothekerzeitung, Jg. 120, Nr. 35,
(1980), S. 1649-1657
Kosmetologie (I), J.S.Jellinek, 2. Auflage
(1967), S. 77
Fette, Seifen, Anstreichmittel 71 (1969),
386-394
"Die Emulsion in der Hauttherapie" (1951),
S.Hirzel Verlag Stuttgart, S.22-23**

(73) Patentinhaber : **SCHERING WIEN Ges.m.b.H.
Scheringgasse 2
A-1140 Wien (AT)**

(72) Erfinder : **Stindl, Wolfgang
Kleinhöfleiner Hauptstr. 24
A-7000 Eisenstadt (AT)**

(74) Vertreter : **Pfeifer, Otto, Dipl.-Ing.
Patentanwälte Schütz und Partner
Fleischmanngasse 9
A-1040 Wien (AT)**

EP 0 065 929 B2

## Beschreibung

Die Erfindung betrifft ein Verfahren zur Herstellung eines kosmetischen Mittels in Form einer Salbe, Paste, Creme oder dergleichen, das hydrophile und/oder lipophile Wirkstoffe, eine Fettphase, eine wässrige Phase, Emulgatoren, Konservierungsmittel sowie Duftstoffe enthält.

Es sind zahlreiche derartige kosmetische Cremes, Salben oder dergleichen, insbesondere sogenannte Nähr- und Feuchtigkeitscremes bekannt, die entweder auf einer Öl/Wasser-Emulsion oder einer Wasser/Öl-Emulsion basieren. Das mit solchen Cremes verbundene Problem liegt allerdings darin, daß die einen unterschiedlichen Fett- und/oder Feuchtigkeitsbedarf aufweisenden Bereiche der Haut bzw. verschiedene Hauttypen beim Auftragen solcher Cremes nicht ihrem speziellen Bedarf entsprechend versorgt werden können. Gemessen am Fett- und/oder Feuchtigkeitsbedarf der Haut wird beim Auftragen solcher Cremes einzelnen Hautbereichen gewöhnlich entweder zu viel oder zu wenig Fettphase, Nährstoffe oder Feuchtigkeit und dergleichen zugeführt. Auch ein nacheinander erfolgendes Auftragen von Cremes auf Basis des einen und dann des anderen Emulsionstyps bringt hier keine Abhilfe.

Aufgabe der Erfindung ist nun ein Verfahren zur Schaffung von kosmetischen Salben, Cremes oder dergleichen, mit deren Hilfe unterschiedliche Hautbereiche gleichzeitig entsprechend ihrem spezifischen Fett-, Nährstoff- und/oder Feuchtigkeitsbedarf versorgt werden können, zu entwickeln.

In der französischen Patentschrift 1 603 602 ein Verfahren zur Herstellung einer Salbe, Creme o. ä. entwickelt, welche eine verzögerte Abgabe eines in ihr enthaltenden Wirkstoffs bewirken soll. Die französische Patentschrift 2 326 914 beschreibt ein Verfahren zur Herstellung einer Wasser/Öl/Wasser-Emulsion. In der japanischen Patentschrift 37 292/70 wird ein Verfahren zur Herstellung einer sehr stabilen wässrigen Aerosollösung beansprucht. All diese Verfahren sind nicht geeignet, um ein kosmetisches Mittel herzustellen, daß die erfindungsgemäße Aufgabe löst.

Die erfindungsgemäße Aufgabe wurde durch Bereitstellung eines Verfahrens zur Herstellung eines kosmetischen Mittels in Form einer Salbe, Paste, Creme oder dergleichen, das einen lipophilen Wirkstoff, eine Fettphase, eine wässrige Phase, Emulgatoren, Konservierungsmittel sowie Duftstoffe enthält, dessen Kennzeichen ist, daß es den lipophilen Wirkstoff, die Fettphase und wässrige Phase in Form einer feinstdispersen Mischung einer einen Öl/Wasser-Emulgator und Konservierungsmittel enthaltenden Öl/Wasser-Emulsion und einer einen Wasser/Öl-Emulgator enthaltenden Wasser/Öl-Emulsion mit einer Teilchengröße von jeweils 2 bis 50 µm enthält, gelöst, das dadurch gekennzeichnet ist, daß man aus Fettphase, wässriger Phase, Öl/Wasser-Emulgator und Konservierungsmittel eine Öl/Wasser-Emulsion und aus Fettphase, wässriger Phase und Wasser/Öl-Emulgator eine Wasser/Öl-Emulsion herstellt und daß man die beiden Emulsionen unter Zusatz von Pur-oba-Öl als lipophilen Wirkstoff unter Vakuum bei einer Temperatur zwischen 20 und 40 °C innig verrührt und darauf die erhaltene feindisperse Mischung mit Duftstoffen versetzt.

Mit Hilfe der die feinst disperse Mischung der beiden Emulsionstypen enthaltenden Creme kann auf der Haut je nach ihrer Feuchtigkeit auf ihren unterschiedlichen Bereichen bzw. je nach Hauttypus ein entsprechender Öl/Wasser- oder Wasser/Öl-Hydrolipidfilm erzeugt werden.

Wenn es auch anscheinend im Hinblick auf die unterschiedlichen Bedürfnisse der Haut als naheliegend angesehen werden könnte, einfach die beiden Emulsionstypen zur Herstellung einer Creme zu vermischen, um so den oben geschilderten Bedürfnissen der Haut zu genügen, so ist diese Vorgangsweise nicht ohne weiteres möglich. Dies deswegen, weil die beiden Emulsionstypen bisher nicht stabil nebeneinander aufrechterhalten werden konnten, sondern vielmehr in Abhängigkeit von den verwendeten Bestandteilen zumindest bald nach der Verarbeitung eine Umwandlung des einen in den anderen Emulsionstyp auftritt, sodaß im Endeffekt nur ein Emulsionstyp in der hergestellten Creme erhalten bleibt.

Wenn nun die beiden Emulsionstypen bei Temperaturen zwischen 20 und 40 °C, vorzugsweise bei 30 °C unter Vakuum besonders schonend, dessen ungeachtet jedoch sehr intensiv, in einen Rührwerksbehälter vereinigt werden, sodaß schließlich eine sehr feine Dispersion der beiden Emulsionen, deren Teilchengröße zwischen 2 und 50 µm, vorzugsweise zwischen 5 und 15 µm beträgt erzielt wird, dann bleiben die beiden Emulsionstypen unverändert nebeneinander in der hergestellten Creme erhalten. Dieses Emulsionssystem bleibt bei erheblicher Verdünnung, z. B. mit Flüssigphase, stabil. Es wird angenommen, daß für die Stabilität dieses Emulsionssystems die Teilchengröße ebenso wie das Zusammenwirken der Emulgatoren von Bedeutung ist. Wesentlich ist, daß bei der Herstellung des erfindungsgemäßen kosmetischen Mittels die zum Einsatz kommenden Emulsionen dispergiert und nicht homogenisiert werden.

Die Vereinigung der beiden Emulsionen erfolgt vorzugsweise unter einem Vakuum von 0,5 torr bis 50 torr. Die Rührgeschwindigkeit ist, wie dem Fachmann wohlbekannt ist, von dem verwendeten Rührwerk abhängig und muß in an sich bekannter Weise ermittelt werden.

Die Öl/Wasser-Emulsionen und die Wasser/Öl-Emulsionen können in konventioneller Weise unter Verwendung konventioneller Emulgatoren hergestellt werden (Kirk Othmer: Enzyclopedia of Chemical Technology, 3. Auflage, 1979; John Wiley & Sons; New York etc. Vol 8, Seite 900 bis 930 und Dr. Otto-Albrecht Neumüller: Römpps Chemie Lexikon 7. Auflage, 1973 ; Franck'sche Verlagshandlung Stuttgart, Seite 1 009 bis 1 013). Die für diese Emulsionen verwendeten Wachse, Emulgatoren und übrigen Zusätze sind die gleichen, wie man sie konventioneller Weise bei emulgierten Hautpflegemitteln verwendet (Dr. Otto-Albrecht Neumüller: Römpps Chemie Lexikon, 7. Auflage 1973; Franck'sche Verlagshandlung Stuttgart, Seite 1 427 und 1 428).

Ein in dem nach dem erfindungsgemäßen Verfahren erhaltenen kosmetischen Mittel eingesetzte Öl/Wasser-Emulsion kann aus einem lipophilen Wirkstoff, Fettphase, Öl/Wasser-Emulgator, wässriger Phase und Konservierungsmittel bestehen.

Als lipophiler Wirkstoff wird Pur-oba-Öl (= Jojoba-Öl) eingesetzt. Als ölige Phase oder als Fettphase in der Öl/Wasser-Emulsion eignen sich Kohlenwasserstoffe wie zum Beispiel Vaseline, Paraffine oder Stearin, oder Wachse wie zum Beispiel Bienenwachs. Geeignete Öl/Wasser-Emulgatoren sind beispielsweise Stearylalkohol, Polyoxyäthylenstearate (wie zum Beispiel MYRJ[(R)]), Komplexemulgatoren (wie zum Beispiel Amphoterin[(R)]) und Sorbitanfettsäureester (wie zum Beispiel Span[(R)]) oder Carboxyvinylpolymerisate (wie zum Beispiel Carbopol[(R)]). Die wässrige Phase kann zusätzlich noch Puffersubstanzen, wie zum Beispiel das Dinatriumsalz der Äthylendiamin-N,N,N',N'-tetraessigsäure und Konservierungsmittel wie Chlorquinaldol, Parabene oder Benzalkoniumchlorid enthalten.

In der Öl/Wasser-Emulsion beträgt der Anteil der inneren Emulsion vorzugsweise 30 bis 49 Gew.%, die Teilchengröße der inneren Emulsion liegt vorzugsweise zwischen 1 μ und 100 μ. Bei der Dispergierung beider Phasen wird sie nochmals zerkleinert und liegt im fertigen Produkt unterhalb von 50 μ.

Ein im nach dem erfindungsgemäßen Verfahren erhaltenen kosmetischen Mittel einsetzbare Wasser/Öl-Emulsion besteht aus Fettphase, Wasser/Öl-Emulgator und wässriger Phase. Als ölige Phase oder Fettphase der Wasser/Öl-Emulsion können Kohlenwasserstoffe, z. B. Paraffine und Vaseline, synthetische, pflanzliche und tierische Öle bzw. Wachse (wie zum Beispiel Olivenöl, Erdnußöl, feines Knochenöl, Mandelöl, Lanolin, Bienenwachs oder Sonnenblumenöl) eingesetzt werden, als wässrige Phase gereinigtes demineralisiertes Wasser und als Wasser/Öl-Emulgator Wollfett (= Lanolin), Fettalkohole wie zum Beispiel Cetylalkohol, Myristylalkohol, Stearylalkohol oder Cerylalkohol, Fettsäureester, wie zum Beispiel Bienenwachs (Cera alba) oder Wachsalkoholester oder Mischester (wie zum Beispiel Dehymuls[(R)]).

In der Wasser/Öl-Emulsion beträgt der Anteil der inneren Emulsion vorzugsweise 30 bis 49 Gew.%, die Teilchengröße der inneren Emulsion liegt vorzugsweise zwischen 1 μ und 100 μ. Bei der Dispergierung beider Phasen wird sie nochmals zerkleinert und liegt im fertigen Produkt unterhalb von 50 μ.

Das Mischungsverhältnis von Öl/Wasser-Emulsion und Wasser/Öl-Emulsion liegt vorzugsweise bei 20 bis 80 Gew.% und insbesondere bei 35 % bis 65 % Öl/Wasser-Emulsion.

Das feindisperse System wird zusätzlich noch mit Duftstoffen, wie zum Beispiel diejenige der Crematest[(R)]-Serie versetzt.

Das nach dem erfindungsgemäßen Verfahren erhaltene kosmetische Mittel in Form einer Nährcreme kann beispielsweise folgende Zusammensetzung haben :

| | | | Toleranzen |
|---|---|---|---|
| Pur-oba-Oil | 5 % | | 5 – 10 % |
| Cera-alba (Bienenwachs) | 1 % | | 1 – 5 % |
| Dehymuls[(R)] | 1 % | | 1 – 3 % |
| Stearylalkohol | 4 % | | 4 – 8 % |
| Kohlenwasserstoffe | 30 % | | 30 – 50 % |
| Carbopol[(R)] | 1 % | | |
| MYRJ[(R)] | 3 % | | 2 – 5 % |
| Dinatriumedetat | 1 % | | |
| Chlorquinaldol | 1 % | | |
| gereinigtes demineralisiertes Wasser | 52 % | | 30 – 55 % |
| Parfumöl | 1 % | | |

Die Angaben sind in Gewichtsprozent aufgeführt.

Das nachfolgende Ausführungsbeispiel dient zur Erläuterung des erfindungsgemäßen Verfahrens, welches mittels allen Vorrichtungen durchgeführt werden kann, die man konventioneller Weise zur Herstellung von Salben, Cremes etc. anwendet.

1. Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB 8 ist die Abkürzung für das Deutsche Arzneibuch, amtliche Ausgabe, 8. Auflage 1978 - 40,00 g Stearylalkohol, 30,00 g MYRJ(R) und 50,00 g Pur-oba-Öl eingetragen. Man rührt die Mischung noch solange, bis eine Emulsion mit einer Teilchengröße von 20-70 µ entsteht.

2. Herstellung der Wasser/Öl-Emulsion 228,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch solange, bis eine Emulsion mit einer Teilchengröße von 20-70 µ entsteht.

3. Herstellung einer Creme. Die Wasser/Öl-Emulsion wird unter kräftigem Rühren unter einem Vakuum von 10 torr in die Öl/Wasser-Emulsion eingetragen. Man rührt noch solange, bis eine Dispersion mit einer Teilchengröße von 10 bis 50 µ entsteht, entfernt das Vakuum und setzt unter Rühren noch 2,00 g eines Duftstoffes der Crematest(R) zu.

## Patentansprüche

1. Verfahren zur Herstellung eines kosmetischen Mittels in Form einer Salbe, Paste, Creme oder dergleichen, das einen lipophilen Wirkstoff eine Fettphase, eine wässrige Phase, Emulgatoren, Konservierungsmittel sowie Duftstoffe enthält, dessen Kennzeichen ist, daß es den lipophilen Wirkstoff, die Fettphase und wässrige Phase in Form einer feinstdispersen Mischung einer einen Öl/Wasser-Emulgator und Konservierungsmittel enthaltenden Öl/Wasser Emulsion und einer einen Wasser/Öl-Emulgator enthaltenden Wasser/Öl-Emulsion mit einer Teilchengröße von jeweils 2 bis 50 µm enthält, dadurch gekennzeichnet, daß man aus Fettphase, wässriger Phase, Öl/Wasser-Emulgator und Konservierungsmittel eine Öl/Wasser-Emulsion und aus Fettphase, wässriger Phase und Wasser/Öl-Emulgator eine Wasser/Öl-Emulsion herstellt und daß man die beiden Emulsionen unter Zusatz von Pur-oba-Öl als lipophilen Wirkstoff unter Vakuum bei einer Temperatur zwischen 20 und 40 °C innig verrührt und darauf die erhaltene feindisperse Mischung mit Duftstoffen versetzt.

## Claims

1. Process for the preparation of a cosmetic preparation in the form of an ointment, paste, cream or equivalent which contains a lipophilic active ingredient, a fat phase, an aqueous phase, emulsifiers, preservative as well as perfumes, whose characteristic is that it contains the lipophilic active ingredient, the fat phase and the aqueous phase in the form of a finely-dispersed mixture of an oil/water emulsion containing an oil/water emulsifier and preservative, and of a water/oil emulsion containing a water/oil emulsifier, with a particle size in each case of from 2 to 50 µm, characterised in that an oil/water emulsion is prepared from the fat phase, aqueous phase, oil/water emulsifier and preservative and that a water/oil emulsion is prepared from the fat phase, aqueous phase and water/oil emulsifier, and that the two emulsions with the addition of pur-oba oil as lipophilic active ingredient are intimately admixed under vacuum at a temperature between 20 and 40°C and the produced finely-dispersed mixture is mixed with perfumes.

## Revendications

1. Procédé pour préparer un produit cosmétique sous la forme d'une pommade, d'une pâte, d'une crème etc., qui contient une substance active lipophile, une phase grasse, une phase aqueuse, des émulsionnants, des agents de conservation et des parfums et dont la particularité essentielle est qu'il renferme la substance active lipophile, la phase grasse et la phase aqueuse sous la forme d'un mélange très finement dispersé d'une émulsion LH ("huile dans l'eau") contenant un émulsionnant LH et un agent de conservation et d'une émulsion HL contenant un émulsionnant HL, dont la dimension particulaire est, pour chacune d'elles, de 2 a 50 µm, procédé caractérisé en ce qu'on prépare une émulsion LH a partir d'une phase grasse, d'une phase aqueuse, d'un émulsionnant LH et d'un agent de conservation et une émulsion HL a partir d'une phase grasse, d'une phase aqueuse et d'un émulsionnant HL, en ce qu'on mélange intimement les deux émulsions, tout en y ajoutant de l'huile pur-oba comme substance active lipophile, sous pression réduite, a une température comprise entre 20 et 40°C, et en ce qu'on ajoute ensuite des parfums au mélange finement dispersé obtenu.